# EUROPEAN PATENT SPECIFICATION

(11) **EP 1 374 898 B1**
(45) Date of publication and mention of the grant of the patent: **27.06.2007**
(21) Application number: 02702914.9
(22) Date of filing: 12.03.2002
(51) Int. Cl.: A61K 45/00, A61K 38/19, A61K 35/28, A61P 25/00

(54) **USE OF INTERLEUKIN-12 FOR TREATING NERVE DAMAGE**
VERWENDUNG VON INTERLEUKIN-12 ZUR BEHANDLUNG VON NERVENSCHÄDEN
UTILISATION DE L'INTERLEUKINE-12 POUR LE TRAITEMENT DES LESIONS NERVEUSES

(30) Priority: 12.03.2001 JP 2001069123; 02.11.2001 JP 2001338772
(43) Date of publication of application: 02.01.2004
(60) Divisional application: 05025861.5
(73) Proprietor: Institute of Gene and Brain Science, Tokyo 160-0015 (JP)
(72) Inventor: TODA, Masahiro, Yokohama-shi, Kanagawa 227-0043 (JP); KAWAKAMI, Yutaka, Yokohama-shi, Kanagawa 221-0861 (JP); TOYAMA, Yoshiaki, Setagaya-ku, Tokyo 158-0097 (JP); MIKAMI, Yuji, Chiyoda-ku, Tokyo 102-0082 (JP)
(74) Representative: Williams, Aylsa
(86) International application number: PCT/JP2002/002310
(87) International publication number: WO 2002/072144

(56) References cited:
- EP-A2- 0 599 303
- WO-A-91/19508
- WO-A-96/24369
- WO-A1-96/06939
- WO-A1-97/09885
- WO-A1-98/41220
- WO-A2-99/16460
- CA-A- 2 213 906
- DATABASE WPI Section Ch, Week 199338 Derwent Publications Ltd., London, GB; Class B04, AN 1993-299557 XP002270738 & JP 05 213767 A (MOCHIDA PHARM CO LTD) 24 August 1993 (1993-08-24)
- DATABASE WPI Section Ch, Week 199328 Derwent Publications Ltd., London, GB; Class B04, AN 1993-224264 XP002270739 & JP 05 148155 A (TORAY IND INC) 15 June 1993 (1993-06-15)
- DATABASE WPI Section Ch, Week 199637 Derwent Publications Ltd., London, GB; Class B04, AN 1996-368120 XP002270740 & JP 08 176008 A (TORAY IND INC) 9 July 1996 (1996-07-09)
- HAIYAN LIN, NAOSHI HIKAWA, TOSHIFUMI TAKENAKA, YOSHIHIRO ISHIKAWA: "Interleukin-12 promotes neurite outgrowth in mouse sympathetic superior cervical ganglion neurons" NEUROSCIENCE LETTERS, vol. 278, no. 3, 14 January 2000 (2000-01-14), pages 129-132, XP002270737
- MIKAMI YUJI ET AL.: 'Sekizui sonsho ni taisuru kogen teiji saibo(jujo saibo) o mochiita men'eki ryoho no kokoromi' THE JOURNAL OF THE JAPANESE ORTHOPAEDIC ASSOCIATION vol. 75, no. 8, 25 August 2001, page S1017, XP002955838

### SEQUENCE LISTING

<110> KEIO UNIVERSITY
<120> Nerve damage drugs
<130> 260PCT
<140>
   <141>
<150> JP 2001/338772
   <151> 2001-11-02
<150> JP 2001/69123
   <151> 2001-03-12
<160> 16
<170> PatentIn Ver. 2.1
<210>
   <211> 21
   <212> DNA
   <213> Artificial Sequence
<220>
   <223> Description of Artificial Sequence:P1
<400> 1
   catggcattg ttaccaactg g 21
<210> 2
   <211> 20
   <212> DNA
   <213> Artificial Sequence
<220>
   <223> Description of Artificial Sequence:P2
<400> 2
   tgtggtggtg aagctgtagc 20
<210> 3
   <211> 22
   <212> DNA
   <213> Artificial Sequence
<220>
   <223> Description of Artificial Sequence:P3
<400> 3
   actacggcaa cagagacgct ac 22
<210> 4
   <211> 22
   <212> DNA
   <213> Artificial Sequence
<220>
   <223> Description of Artificial Sequence:P4
<400> 4
   acaggctctc actgtcacac ac 22
<210> 5
   <211> 21
   <212> DNA
   <213> Artificial Sequence
<220>
   <223> Description of Artificial Sequence:P5
<400> 5
   tggctagcaa ggaagattcg t 21
<210> 6
   <211> 21
   <212> DNA
   <213> Artificial Sequence
<220>
   <223> Description of Artificial Sequence:P6
<400> 6
   acggaggtca tggatagacc t 21
<210> 7
   <211> 20
   <212> DNA
   <213> Artificial Sequence
<220>
   <223> Description of Artificial Sequence:P7
<400> 7
   tgctggtgac aaccacggcc 20
<210> 8
   <211> 20
   <212> DNA
   <213> Artificial Sequence
<220>
   <223> Description of Artificial Sequence:P8
<400> 8
   gtactccaga agaccagagg 20
<210> 9
   <211> 20
   <212> DNA
   <213> Artificial Sequence
<220>
   <223> Description of Artificial Sequence:P9
<400> 9
   gaagccatcc gtggccagat 20
<210> 10
   <211> 20
   <212> DNA
   <213> Artificial Sequence
<220>
   <223> Description of Artificial Sequence:P10
<400> 10
   gacgtcaaaa gacagccact 20
<210> 11
   <211> 21
   <212> DNA
   <213> Artificial Sequence
<220>
   <223> Description of Artificial Sequence:P11
<400> 11
   acagccctga agtggataga g 21
<210> 12
   <211> 21
   <212> DNA
   <213> Artificial Sequence
<220>
   <223> Description of Artificial Sequence:P12
<400> 12
   gggcttcagc atgctgcctt g 21
<210> 13
   <211> 21
   <212> DNA
   <213> Artificial Sequence
<220>
   <223> Description of Artificial Sequence:P13
<400> 13
   ccagcagaaa gagtagagga g 21
<210> 14
   <211> 21
   <212> DNA
   <213> Artificial Sequence
<220>
   <223> Description of Artificial Sequence:P14
<400> 14
   atgaaagaag taaacgtcca c 21
<210> 15
   <211> 22
   <212> DNA
   <213> Artificial Sequence
<220>
   <223> Description of Artificial Sequence:P15
<400> 15
   gttttggcct gtggtcgtgc ag 22
<210> 16
   <211> 22
   <212> DNA
   <213> Artificial Sequence
<220>
   <223> Description of Artificial Sequence:P16
<400> 16
   gcgcttgctc cggtgagtcc tg 22

## Description

### Technical Field

The present invention relates to a remedy for a nerve dysfunctional disorder such as a central nervous system damage including a spinal cord injury and a cerebral infarction and the like which promotes nerve regeneration, or more particularly, a remedy which can be applied to gene therapies.

### Background Art

Most spinal cord injuries are traumatic, and their causes are traff ic accidents, sports, industrial accidents and the like, whereas the causes of atraumatic injuries are inflammation, bleeding, tumor, spinal deformation and the like. Their pathological features are crush of a spinal cord and a compression lesion with bleeding and edema in spinal parenchyma as a basal plate, and a neuropathy corresponding to a injured site occurs. As a main clinical symptom, incompetent or competent motor palsy and numbness occur on and under the level of injury, and for cervical spinal cord injury, respiratory palsy and hyperpyrexia (or severe hypothermia) can be seen as distinctive complications. Improvement of the aforementioned neuropathy, particularly the improvement of dyskinesia is directly linked to the inhibition of increment in bedridden old people and the progress of QOL (Quality of Life), and therefore, their importance is growing in parallel with the extension of average life expectancy in these years.

Therapies being conducted for the aforementioned spinal cord injury are surgical operations for eliminating physical compression and injuries, and steroid therapies for a spinal cord edema at the acute stage of injury (N. Engl. J. Med. 322, 1405-1411, 1990; J. Neurosurg 93, 1-7, 2000). Among the steroidal agents, it is reported that megadoses of methylprednisolone are effective for the improvement of neurological symptom associated with a spinal cord injury (J. Spinal Disord. 5(1), 125-131, 1992), however, there is a problem, in megadoses of steroidal agents, of lowering the phylactic function in the case of the spinal cord injuries which are associated with infection, in addition to the strong expression of systematic adverse reactions and the difficulty in controlling them. Besides, even the efficacy of steroid-megadosed therapies remains controversial for the present. As described above, there has been no effective remedy for a spinal cord injury to date, therefore it has been aspired for the development of a new remedy. Other therapeutic methods for spinal cord injuries reported in addition to the aforementioned are as follows: a method wherein therapeutically effective amount of glioastrocytoma which was pretreated by inflammation related cytokine in vitro is transplanted to the injured site in the central nervous system (CNS) (Published Japanese translation of PCT International Publication No.2000-503983); a method wherein regeneration of a neurological axon in the central nervous system (CNS) of mammal animals is promoted by administering congeneric monocular macrophages (monocytes, macrophages, etc.) to the injured site or disordered site, or CNS of its vicinity (J. Mol. Med. 77, 713-717, 1999; J. Neurosci. 19(5), 1708-16, 1999; Neurosurgery 44(5), 1041-5, 1999, Trends. Neurosci 22(7), 295-9, 1999) (Published Japanese translation of PCT International Publication No.H11-13370) and the like. Further, although the defined mechanism is uncertain, it is also reported that restoration of motion sustainment after a spinal cord injury was promoted by the vaccination of spinal cord homogenate and administering a T cell specific to a myelin basic protein which is a myelin protein (Neuron 24, 639-647, 1999; Lancet 354, 286-287, 2000).

On the other hand, dendritic cells (DC) are the cell population taking dendritic forms that are derived from hematopoietic stem cells, and are widely distributed in vivo. Immature dendritic cells undertake a role as antigen-presenting cells that induce immunoresponse by activating antigen-specific T cells, by way of recognizing and incorporating a foreign body such as a virus and a bacterium which has invaded each tissue, generating a peptide by digesting and degrading such foreign body in the process of transfer to a lymphatic organ T cell region, binding such peptide to a MHC molecule, and presenting such peptide to the cell surface (Ann. Rev. Immunol. 9, 271-296, 1991; J. Exp. Med. 185, 2133-2141, 1997).

It had been difficult to prepare a large quantity of dendritic cells due to their low-density in each tissue despite that they are widely distributed, however, it became possible to easily prepare a large quantity of such cells in vitro by adding differentiated growth factors to the culture of immature precursor cells. Therefore, it has been started to consider using dendritic cells as immunostimulator (J. Exp. Med. 183, 7-11, 1996). It is particularly targeted to specifically enhance the immunoresponse by pulsing antigens to dendritic cells against a faint tumor immunoresponse. In an animal experiment, it is shown that dendritic cells presenting a protein and an antigen peptide derived from a tumor induce a specific CD8⁺ cytotoxic T cell. It is reported also in human that tumors decreased or disappeared by returning a protein and an antigen peptide derived from a tumor together with dendritic cells to a living body. Meanwhile, it is reported that IL-12, a cytokine, is secreted mainly from the antigen-presenting cells such as the aforementioned dendritic cells and B cells, and acts toward T cells and NK-cells, and has a high antitumor activation (J. Exp. Med. 178, 1223-1230, 1993; J. Exp. Med. 189, 1121-1128, 1999). Thus, IL-12 draws attention as a remedy for cancer, and clinical trials have been conducted as a new immunotherapy for cancer. However, it has not historically been applied for a nervous system at all.

On the other hand, one of the most important elements in the study of spinal cord injury wherein an animal model is used can be exemplified by the evaluation of motor function. Such evaluation of motor function is desired to be easy and to have high reproducibility. However, most of the historical evaluation methods of motor function emphasize the movements of articulations of individual posterior limbs and their coordinated movements or the overall conditions of locomotion, as in the BBB scoring method (J Neurosung 93, 266-75, 2000) wherein the locomotion of animals are evaluated by the total scores (the maximum score is 21 points) of various check items, and even including the one requiring detailed measurement of the motion which were videotaped in advance. Therefore, there was a problem that such methods were cumbersome and might easily cause individual variations among the experimenters.

Injuries of central nervous systems including spinal cord injuries are disorders, which are extremely difficult to be remedied, and there has been no effective therapy to date as described above, therefore, the development of a new therapy is strongly expected. In addition, the number of patients affected by nervous system disorders is on the rise in connection with the aging of population, and it has become a major social problem. However, the central nervous system is an organ, which is extremely difficult to be regenerated, and is a special organ wherein immunoreaction is hard to occur. In the aforementioned method by Schwartz et al. wherein regeneration of nervous axon in the central nervous system (CNS) is promoted by using macrophages, it was not clear which function of the macrophages prompts the regeneration of an axon. When the cells such as macrophages and the like are used, there were the problems not only that the administration method was limited, but also that its handling was complicated, and that it is hard to obtain reproducible therapeutic effect since a living cell was used. The object of the present invention is to provide a remedy for a nerve dysfunctional disorder such as a central nervous system injury including a spinal cord injury and a cerebral infarction, which can be administered not only by injecting into a injured site but also by various administration methods including subcutaneous administration, administration to a vicinity of lymph nodes, and intravenous administration, which can easily be handled and stored over a long time, and can be prepared in a large quantity at any time, and containing distinguished nerve regeneration promotion action.

Unlike other tissues, the central nervous system is a tissue that is isolated from the immune system. However, the present inventors recently reported that immature T cells which are not stimulated at all can not invade into the central nervous system, however, T cells activated by an antigen in a brain can pass through a blood brain barrier and can be reacted with a brain tumor, as a result of experiment wherein a mouse brain tumor model was used (Neuro-Oncology 1, S105, 1999). In addition, there is a report that restoration of central nerve damage was promoted by administering nervous specific T cells (Lancet 354, 286-287, 2000). It is still uncertain how the nervous specific T cells function in the central nervous system after passing through the blood brain barrier, for example, whether by releasing some sort of cytokine or whether they act by directly attaching to a nervous cell or an axon or any other, however, the possibility of nerve regeneration by an intervention of immune system is indicated. Meantime, it is necessary to incorporate an antigen of a nervous system by an antigen-presenting cell, and to present an antigen peptide treated within the cell to T cells in order to induce a nervous specific T cell.

The present inventors have substantiated for the first time that exclusion of the injured tissue at the time of spinal cord injury is the first phase of crucial importance, and that restoration of spinal cord function is promoted by incorporating an antigen and directly transplanting certain dendritic cell subsets having the highest antigen presenting ability against T cells to the injured site of the spinal cord injured model mouse. For the aforementioned substantiation of promoting restoration of spinal cord function, the evaluation method for motor function in the spinal cord injured mouse established by the present inventors was used. In this evaluation method for motor function, an apparatus which was used for measuring the amount of motion for the purpose of analyzing sedative effects and the like of a drug is applied to the evaluation of motor function after a spinal cord is injured. The present inventors targeted a substance secreted from dendritic cells generating environmental changes including activation of T cells in the central nervous system, or a substance inducing and proliferating, or activating dendritic cells, and the present inventors administered such candidate substance to the injured site of a spinal cord injured model mouse, and screened by the aforementioned evaluation method for motor function in the spinal cord injured mouse, and as a result, the present inventors found that IL-12 which are widely used as remedies for cancer but are not used for nervous system at all, and GM-CSF promote restoration of spinal cord function as dendritic cells do. Besides, as described above, since significant restoration of motor function was recognized by transplanting dendritic cell subsets into the injured spinal cord, the present inventors analyzed a substance promoting the nerve regeneration which are secreted from dendritic cells, and confirmed that such dendritic cells express a neurotrophic factor, and actually secrete the same. The present invention has been completed as a result of these findings.

### Disclosure of the Invention

The present invention relates to: a remedy for a nerve damage or a nerve dysfunctional disorder wherein
an interleukin (IL)-12 is used as an active ingredient.

Taught herein for reference is a remedy for a nerve damage or a nerve dysfunctional disorders wherein the substance secreted from dendritic cells, the substance inducing and proliferating dendritic cells, the substance activating dendritic cells, the substance inducing the expression of a neurotrophic factor in nerve tissues, and the substance inducing and proliferating microglias and macrophages in nerve tissues are cytokines; a remedy for a nerve damage or a nerve dysfunctional disorders wherein the cytokine inducing and proliferating dendritic cells is a granulocyte-macrophage colony-stimulating factor (GM-CSF); a remedy for a nerve damage or a nerve dysfunctional disorder wherein the cytokine inducing the expression of a neurotrophic factor in nerve tissues is a granulocyte-macrophage colony-stimulating factor (GM-CSF); a remedy for a nerve damage or a nerve dysfunctional disorder wherein the cytokine inducing and proliferating mioroglias and macrophages in nerve tissues is a granuloeyte-macrophage colony-stimulating factor (GM-CSF).

The present invention further providers use of a vector which can express IL-12 for preparing a remedy for a nerve damage or a nerve dysfunctional disorder.

Also disclosed herein for reference are: a remedy for a nerve damage or a nerve dysfunctional disorder wherein the dendritic cells are dendritic cell subsets secreting a neurotrophic factor NT-3; a remedy for a nerve damage or a nerve dysfunctional disorder wherein the dendritic cell subsets secreting a neurotrophic factor NT-3 are immature dendritic cell subsets expressing CNTF, TGF-β 1, IL-6 in addition to NT-3, or mature dendritic cell subsets expressing CNTF, TGF-β 1, IL-6, EGF in addition to NT-3; a remedy for a nerve damage or a nerve dysfunctional disorder wherein the dendritic cell subsets secreting a neurotrophic factor NT-3 are immature dendritic cell subsets having a surface marker of CD11c on the cell surface, or mature dendritic cell subsets derived from said immature dendritic cells; a remedy for a nerve damage or a nerve dysfunctional disorder wherein the mature dendritic cell subsets are mature dendritic cell subsets which can be obtained by culturing immature dendritic cell subsets in vitro under the presence of a stimulating agent aimed for maturing immature dendritic cells; a remedy for a nerve damage or a nerve dysfunctional disorder wherein the mature dendritic cell subsets are mature dendritic cell subsets wherein a protein or a peptide of a nervous system, or an expression system of a gene that encodes them is introduced; a therapy method for a nerve damage or a nerve dysfunctional disorder wherein the remedy for a nerve damage or a nerve dysfunctional disorder is administered to a nerve injured site, subcutaneously, to a vicinity of lymph nodes, or intravenously.

### Brief Description of Drawings

Fig. 1 is a set of drawings showing the result of evaluation of motor function of spinal cord injured model BALB/c mouse.
Fig. 2 is a set of drawings showing the result of evaluation of motor function of spinal cord injured model C57BL/6 mouse.
Fig. 3 is a drawing showing the effect of antigen-presenting cells including dendritic cells for a spinal cord injury.
Fig. 4 is a drawing showing the effect of dendritic cells of CD11c (+) for a spinal cord injury.
Fig. 5 is a drawing showing the effect of IL-12 of the present invention for a spinal cord injury.
Fig. 6 is a drawing showing the effect of GM-CSF of the present invention for a spinal cord injury.
Fig. 7 is a drawing showing the result of expression of neurotrophic factor in immature dendritic cell subsets by RT-PCR.
Fig. 8 is a drawing showing the result of expression of neurotrophic factor in mature dendritic cell subsets by RT-PCR.
Fig. 9 is a drawing showing the result of secretion of NT-3 such as dendritic cells and the like by ELISA.
Fig. 10 is a set of drawings showing the effect of dendritic cell subsets secreting a neurotrophic factor for a spinal cord injury.
Fig. 11 is a set of photographs chronologically showing a representative section particularly from marginal injured site to cephalad direction as a result of immunostaining by using anti-Mac-1 antibody in each of the dendritic cells (DC) and the RPMI1640 (RPMI) transplanted group.
Fig. 12 is a drawing showing the chronological change in the number of Mac-1 positive ameboid cells by each region in each of the dendritic cells and the RPMI1640 transplanted group.
Fig. 13 is a drawing showing the chronological change in the number of Mac-1 positive ramified cells by each region in each of the dendritic cells and the RPMI1640 transplanted group.
Fig . 14 is a set of photograph showing the setting of regions for measuring the number of Musashi-1 positive cells.
Fig. 15 is a set of photographs chronologically showing a representative section particularly from marginal injured site to cephalad direction as a result of immunostaining by using an anti-Musashi-1 antibody in each of the dendritic cells (DC) and the RPMI1640 (RPMI) transplanted group.
Fig. 16 is a set of drawing showing the chronological change in the number of Musashi-1 positive cell by each region in each of dendritic cells and RPMI1640 transplanted group.
Fig. 17 is a drawing showing the result of expression of a neurotrophic factor in a spinal cord injured site after the administration of GM-CSF by RT-PCR.
Fig. 18 is a drawing showing the setting of regions for measuring the number of Mac-1 positive cells.
Fig. 19 is drawing showing the chronological change in the number of endogenous microglia cells (ameboid) in each of the GM-CSF administered group and a control (physiological saline administered) group.
Fig. 20 is a drawing showing the chronological change in the endogenous microglia cells (ramified) in each of the GM-CSF administered group and a control (physiological saline administered) group.
Fig. 21 is a drawing showing the setting of regions for measuring the number of Musashi-1 positive cells.
Fig. 22 is a drawing showing the chronological change in the number of Musashi-1 positive cells in each of the GM-CSF administered group and a control (physiological saline administered) group.

### Best Mode of Carrying out the Invention

The remedy for a nerve damage or a nerve damage or a nerve dysfunctional disorder of the present invention can be exemplified by IL-12.

Also disclose herein for reference is a remedy for a nerve damage or a nerve dysfunctional disorder exemplified by the following: a substance secreted from dendritic cells; a substance inducing and proliferating dendritic cells; a substance activating dendritic cells; a substance inducing the expression of a neurotrophic factor in nerve tissues; a substance inducing and proliferating microglias and macrophages in nerve tissues, wherein the substances have an effect of prevention, symptom improvement or a therapeutic effect for a nervous injury or a nerve dysfunctional disorder (these substances will be collectively referred to as a "dendritic cell related active substance" hereinafter), or a mixture of these substances that are used as active ingredients. Said substance secreted from the aforementioned dendritic cell can be eligibly exemplified by cytokines such as IL-1α, IL-1β, IFN-γ and the like, said substance inducing and proliferating dendritic cells can be eligibly exemplified by cytokines such as GM-CSF, IL-4 and the like, and said substance activating dendritic cells can be eligibly exemplified by IL-1*β*, CD40L and the like. Said substance inducing the expression of a neurotrophic factor in nerve tissues after the injury can be eligibly exemplified by cytokines such as GM-CSF and the like, and said substance inducing and proliferating microglias and macrophages in nerve tissues after the injury can be eligibly exemplified by cytokines such as GM-CSF, M-CSF and the like. The above-mentioned neurotrophic factor can be exemplified by NT-3 inducing the effect of nerve regeneration in vivo, the proliferation of microglias, and the enhancement of phagocytosis; BDNF inhibiting denaturation and omission of motor neuron of the injured spinal cord; NGF being a neurotrophic factor of cholinergic neuron; CNTF having the effects of denaturation and cell death protection against both motor and sensory neurons of spinal cord, and the like.

Each known substance having the inducing and proliferating action and the like of dendritic cells can be used as the following substances: a substance secreted from dendritic cells; a substance inducing and proliferating dendritic cells; a substance activating dendritic cells; a substance inducing the expression of a neurotrophic factor in nerve tissues; and a substance inducing and proliferating microglias and macrophages in nerve tissues. For example, said substance secreted from dendritic cells can be obtained by culturing dendritic cells in vitro; said substance having the inducing and proliferating action of dendritic cells can be obtained by culturing dendritic cells under the presence of a candidate substance in vitro, and measuring and evaluating the extent of the induction and proliferation of dendritic cells; said substance activating dendritic cells can be obtained by culturing dendritic cells under the presence of a candidate substance in vitro and measuring and evaluating the extent of neurotrophic factor generation ability of dendritic cells; said substance inducing the expression of a neurotrophic factor in nerve tissues can be obtained by measuring and evaluating the extent of the expression and induction of a neurotrophic factor in injured neural tissues wherein a candidate substance is administered. Said substance inducing and proliferating microglias and macrophages in nerve tissues can be obtained by measuring and evaluating the extent of the induction and proliferation of the following cells in injured neural tissue wherein a candidate substance is administered: ameboid cells, in the injured neural tissues wherein a candidate substance is administered, considered to be the activated microglias with the strong phagocytic capacity and macrophages derived from monocytes flown from the outside of spinal cord; ramified cells considered to be activated microglias secreting various neurotrophic factors and cytokines though being lack of phagocytic capacity.

In the case where the aforementioned dendritic cell related active substance is used as a remedy for a nerve damage or a nerve dysfunctional disorder, various compound ingredients for dispensing such as an ordinary carrier that is pharmaceutically tolerated, a bonding agent, a stabilizing agent, an excipient, an diluent, a pH buffer agent, a disintegrant, a solubilizer, a dissolution coadjuvant, an isotonic agent and the like can be added. Said remedy can be administered orally or parenterally. More specifically, it can be administered orally by ordinary administering formulations such as formulations of powders, granules, capsules, syrups, and liquid suspension, or it can also be administered parenterally to the spot by injecting the formulations of solution, emulsion, liquid suspension and the like, or it can be further administered through the nostril by the formulation of a spray agent.

In addition, as the aforementioned dendritic cell related active substance, a vector which can express said substance can be used, and when said vector is administered to the spot as a genetic therapy, it becomes possible to provide a dendritic cells related active substance to the spot stably due to the stable expression of said substance compared to the case wherein a remedy containing a dendritic cells related active substance as an active ingredient is administered to the spot. In contrast to the fact that most of the dendritic cells related active substance of which the half-life periods are extremely short and unstable, stable expression during the specified time can be obtained by transferring a gene into a cell at the nerve injured site with the use of a vector which can express a dendritic cells related active substance. Such vectors can be eligibly exemplified by virus vectors such as herpesvirus (HSV) vectors, adenovirus vectors, human immunodeficiency virus (HIV) vectors and the like, however, HSV vectors are preferable among these virus vectors. HSV vectors have a high nervous affinity and are safe since HSV is not integrated into chromosome DNA of cells, and it is possible to regulate the expression period of a transgene. In addition, virus vectors that express a dendritic cells related active substance can be prepared by ordinary protocols.

Further, a remedy for a nerve damage or a nerve dysfunctional disorder can be exemplified by the one comprising dendritic cells, or particularly preferably, dendritic cell subsets secreting a neurotrophic factor NT-3 as an active ingredient. As for the aforementioned dendritic cell subsets secreting the neurotrophic factor NT-3,the following subsets are preferable: immature dendritic cell subsets expressing the following, CNTF showing the effects of denaturation and cell death protection against both motor and sensory neurons of the spinal cord, TGF-β1 having an inhibitory action for releasing cytotoxic substance derived from microglias and macrophages, and IL-6 inducing the protection effect for various neurons (cholin catecholamine dopaminergic), in addition to NT-3 inducing the nerve regeneration effect in vivo, the proliferation of microglias, and the enhancement of phagocytosis; mature dendritic cell subsets expressing CNTF, TGF-β 1, IL-6 and EGF wherein the nervous protection effect is acknowledged, in addition to NT-3. Such subsets can be exemplified by immature dendritic cell subsets having a surface marker of CD11c on the cell surface, and mature dendritic cell subsets derived from said immature dendritic cells.

As the aforementioned mature dendritic cell subsets, a mature dendritic cell subsets , which can be obtained by culturing immature dendritic cell subsets in vitro under the presence of a stimulating agent for maturing immature dendritic cells such as LPS, IL-1, TNF-α, CD40L and the like, can be used. In this case, there is a possibility that higher regeneration effect can be induced due to the change in expression of neurotrophic factor such as NT-3 and the like. Besides, mature dendritic cell subsets wherein expression systems of myelin proteins such as MBP (myelin basic protein), MAG (myelin-associated glycoprotein) and the like, proteins and peptides of a nervous system of inhibitors for the extension of nervous axon such as Nogo and the like, or virus vectors wherein the genes encoding them are integrated, are introduced (incorporated) can also be used.

Dendritic cell subsets secreting a neurotrophic factor NT-3 can be obtained by, for example, separating dendritic cell subsets by a method wherein peripheral blood and the like are pretreated by a density centrifugation and the like, then sorted by FACS with the use of a monoclonal antibody against dendritic cell surface antigen, or by a separation method wherein a magnetic beads binding monoclonal antibody against dendritic cells surface antigen, then by selecting dendritic cell subsets secreting NT-3 from said subsets. Said dendritic cell subsets secreting neurotrophic factor NT-3 can be transplanted to a nerve injured site of spinal cord and the like. Besides, mature dendritic cell subsets wherein the expression system of a protein or a peptide of the aforementioned nervous system, or the genes encoding them is introduced (incorporated) can be administered subcutaneously, or to a vicinity of lymph nodes. As described above, a therapy method for a nerve damage or a nerve dysfunctional disorder can be exemplified by the method wherein a remedy for a nerve damage or a nerve dysfunctional disorder wherein a homogenous dendritic cell subset secreting the aforementioned dendritic cell related active substance or a neurotrophic factor NT-3 as an active ingredient is administered (transplanted) to the nerve injured site, subcutaneously or to a vicinity of lymph nodes, or intravenously.

The present invention will be further specifically explained in the following examples, but the technical scope of the invention will not be limited to these examples.

### Example 1 (Generation of Spinal Cord Injured Model BALB/c Mouse)

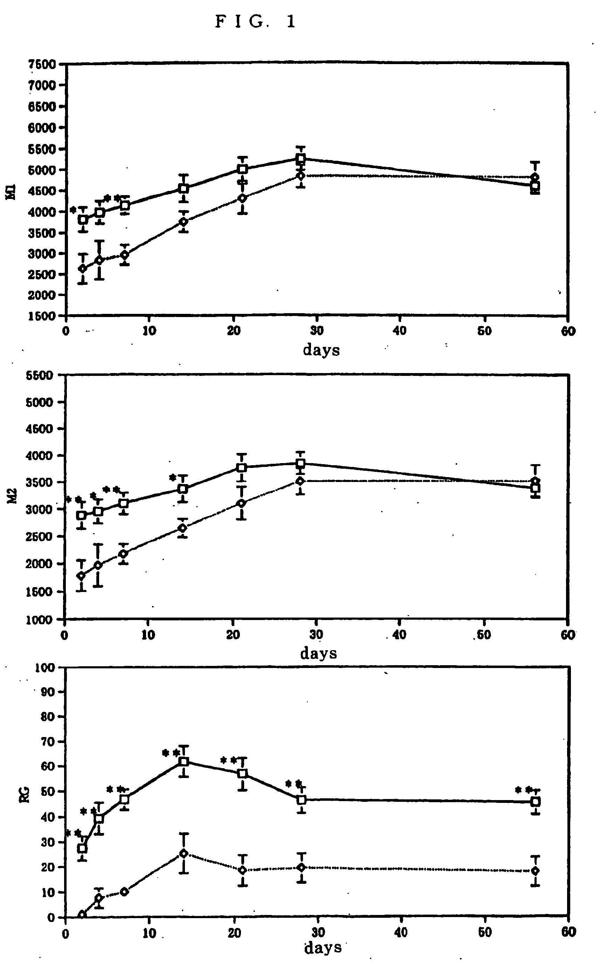

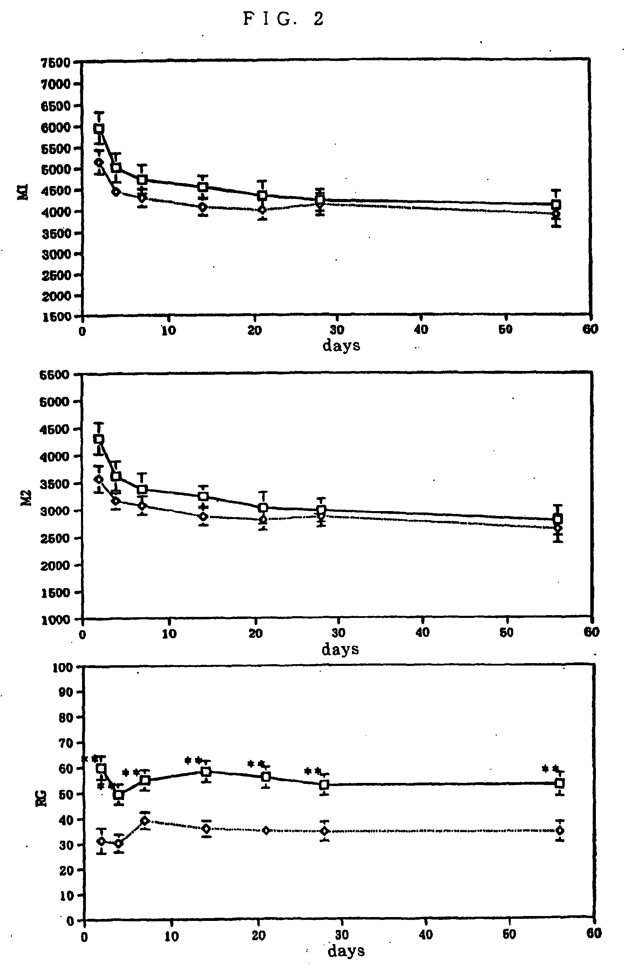

BALB/c female mice (n = 9) of 6 weeks old were used respectively, the eighth thoracic vertebra was laminectomized under ether anesthesia, and the left side of the spinal cord was cut half by a sharp blade, and spinal cord injured model mice (injured group; ◇) were generated. After the spinal cord was injured, these mice showed palsy in the left lower limbs. A group of BALB/c female mice of 6 weeks old (n = 9) wherein only laminectomy was conducted were used as a control (control group; □). The amount of spontaneous motion of each of the aforementioned mice were measured by using an action analyzing apparatus SCANET MV-10 (Toyo Sangyo; an apparatus wherein 144 sets of near infrared radiation sensors running in all directions are installed two-tiered in a square of 426 mm square) and motor function was evaluated after the surgery, on day 2 and 4 as in the acute stage, day 7 as the subacute stage, day 14, 21, 28, and 56 as the chronic stage. In addition, measurement of spontaneous motor quantity was set to detect and measure in forms of two sizes of horizontal movements (Movement 1, 2; M1, M2 for abbreviation, it is regarded that a motion is made and the motor quantity is measured when the motion was recognized in 12 mm or more for M1 and in 60 mm or more for M2), and vertical movements (Rearing; RG for abbreviation, the number of uprising motion of 6.75 cm or more is measured), and it was further set to measure for 10 minutes per mouse. The result of the case wherein BALB/c female mice were used is shown in Fig. 1. Besides, the p value in the figure was calculated by using the Student's t test (*: p < 0.05, **: p < 0.01). As a result of comparing the evaluation of each motor function between a control group and the injured group, in M1 (upper stand of Fig. 1) and M2 (middle stand of Fig. 2) which show the evaluation of horizontal movements, a significant difference was recognized in the acute and subacute stage, however, significant difference was not recognized in the chronic stage. On the other hand, in RG that shows the evaluation of vertical movements, an obviously significant difference was recognized between both groups until the chronic stage (the lower stand of Fig. 1).

### Example 2 (Generation of Spinal Cord InjuredModel C57BL/6 Mouse)

With the exception of using C57BL/6 female mice of 6 weeks old (n = 16) instead of the aforementioned BALB/c female mice of 6 weeks old (n = 9), evaluation of motor function was conducted with the use of the action analyzing apparatus SCANET MV-10 in the same manner as in Example 1. The result is shown in Fig. 2. The p value in the figure was calculated by using the Student's t test (**: p < 0.01). As a result of comparing the evaluation of each motor function between a control group (□) and the injured group (◇), an obviously significant difference was not recognized in M1 which shows the evaluation of horizontal movements (upper stand in Fig. 2) and M2 (middle stand in Fig. 2) throughout the acute, subacute, and chronic stage. On the other hand, in RG that shows the evaluation of vertical movements, an obviously significant difference was recognized in both groups until the chronic stage (lower stand in Fig. 2). The results of the aforementioned experiments between two different types of mice in different strains showed that in vertical movements (RG), it is possible to precisely evaluate the motor function after the spinal injury, in contrast to the case of the amount of horizontal movements (M1 and M2) wherein it was compensated by a lower limb on the unaffected side or both upper limbs, and it was impossible to precisely evaluate the palsy in the light lower limb.

### Example 3 (The Effect of Dendritic Cells against Spinal Cord Injury)

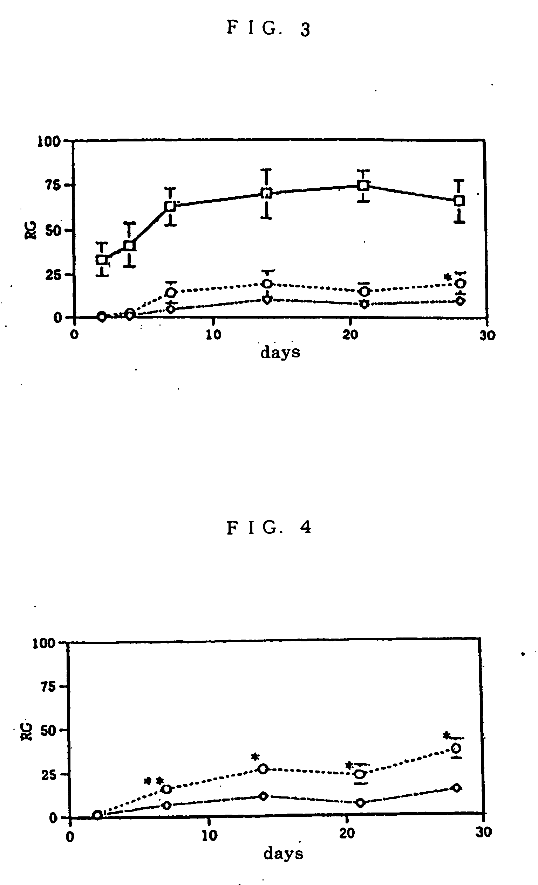

Spinal cord injured model mice (BALB/c female mice) were generated in the same operation as in Example 1, and immediately thereafter, only RPMI1640 culture medium [control (◇), Fig. 3; n = 14, Fig. 4; n = 6] or antigen presenting cells including dendritic cells isolated from the spleen [5 x 10⁵/mouse, n = 13, (Fig. 3; ○)], or dendritic cells obtained by sorting a subset of CD11c (+) by applying the immunomagnetic beads method [1 x 10⁵/mouse, n = 6, (Fig. 4 ; ○)] was transplanted to the spinal cord injured site. Besides, mice wherein only a laminectomy was conducted were used as a control of which spinal cord is not injured [Fig. 3 ; □ (n = 6)]. As in Example 1, the amount of spontaneous vertical movement of each mouse were measured by using the action analyzing apparatus SCANET MV-10 and the motor function was evaluated on day 2, 4, 7, 14, 21, 28, and 56. Those results are shown in Fig. 3 and Fig. 4 . In addition, the p value in the figures was calculated by using the Student's test (*: p < 0. 05, **: p < 0.01). These results showed that a significant difference was recognized in the amount of vertical movement compared to a control by administering CD11c (+) dendritic cell subset to the injured site. As a result of the aforementioned, it was revealed that restoration of the spinal cord function is promoted by administering dendritic cells to the nerve injured site.

### Example 4 (The Effect of IL-12 against Spinal Cord Injury)

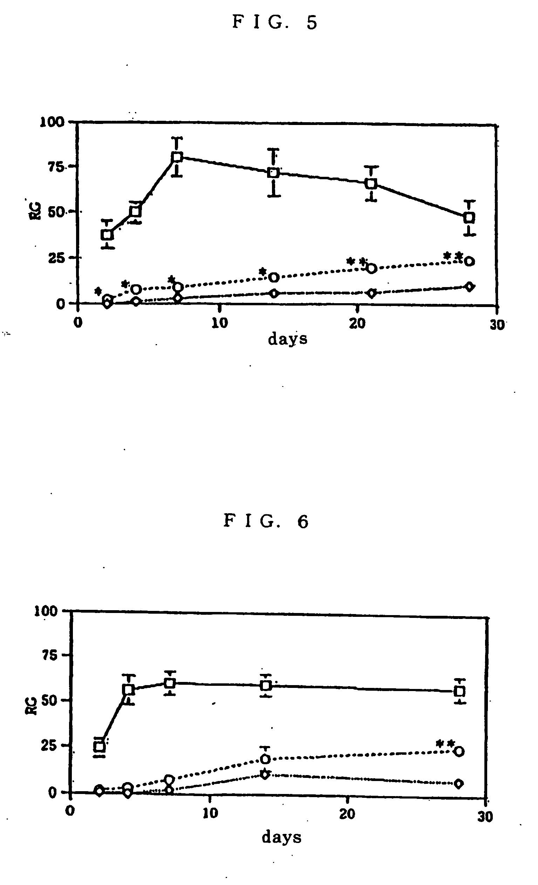

Spinal cord injured model mice were generated by conduct ing the same operation as in Example 1 to the BALB/c female mice of 6 weeks old. Besides, BALB/c female mice of 6 weeks old (□; n = 6) wherein only a laminectomy was conducted were used as a control of which spinal cord was not injured. 5 µl of physiological saline only (◇ ; n = 14) or IL-12 (100 ng/mouse; Pharmingen ; ○ ; n = 14) was administered to the spinal cord injured site immediately after the spinal cord was injured, and then the amount of spontaneous vertical movements of each mouse were measured by using the action analyzing apparatus SCANET MV-10 and the motor function was evaluated on day 2 , 4 , 7 , 14, 21, and 28 as in Example 1. The result is shown in Fig. 5. In addition, the p value in the figure was calculated by using the Student's t test (*: p < 0.05, **: p < 0.01). These results showed that an obviously significant difference was recognized in the amount of vertical movements by administering IL-12 to the injured site compared to the administration of physiological saline. As a result of the aforementioned, it was revealed that restoration of the spinal cord function is promoted by administering IL-12 to the nerve injured site as in the case of using dendritic cells mentioned above.

### Example 5 (The Effect of GM-CSF for Spinal Cord Injury)

Spinal cord injured model mice were generated by conducting the same operation as in Example 1 to the BALB/c female mice of 6 weeks old. Besides, BALB/c female mice of 6 weeks old (□; n = 6) wherein only a laminectomy was conducted were used as a control of which spinal cord was not injured. 5 µl of physiological saline only (◇ ; n = 7) or GM-CSF (10ng/mouse; Genzyme ; ○ ; n = 6) was administered to the spinal cord injured site immediately after the spinal cord was injured, and then the amount of spontaneous vertical movements of each mouse were measured by using the action analyzing apparatus SCANET MV-10 and the motor function was evaluated on day 2, 4, 7, 14, 21, and 28 as in Example 1. The result is shown in Fig. 6. In addition, the p value in the figure was calculated by using the Student's t test (**: p < 0.01). These results showed that an obviously significant difference was recognized in the amount of vertical movement by administering GM-CSF to the injured site compared to the administration of physiological saline. As a result of the aforementioned, it was revealed that restoration of the spinal cord function is promoted by administering GM-CSF to the nerve injured site as in the case of using dendritic cells mentioned above.

### Example 6 (Preparation of Immature Dendritic Cell Subsets and Mature Dendritic Cell Subsets)

Immature dendritic cells were obtained by separating CD11c positive subsets from the spleen of BALB/c female mature mice of 6 weeks old by applying immune magnetic beads method. More precisely, the cell was separated as follows: the spleen was firstly homogenated in 100 U/ml collagenase (Worthington Biochemical Corporation), then coated part which is hard to be separated was incubated in 400 U/ml collagenase at 37° C under 5% CO₂ for 20 minutes. The cells obtained herein were floated in 35% BSA solution, and the RPMI1640 + 10% embryonic sera were stratified in a centrifuge tube, centrifuged at 4° C, 3000 rpm, for 30 minutes, then the cells at the boundary area between 35% BSA solution and the RPMI1640 + 10% embryonic sera solution were collected. Next, the cells obtained herein were reacted with magnetic beads-bound monoclonal antibodies against CD11c antigens (2 x 10⁸ beads, Miltenyi Biotec) at 4°C for 15 minutes, and beads-bound cells were separated by magnets, and thus fractions wherein immature dendritic cell subsets were condensed were obtained. In addition, mature dendritic cell subsets were obtained by culturing the immature dendritic cell subsets obtained in the RPMI1640 + 10% embryonic sera culture solution at 37° C, under 5% CO₂ for 24 hours.

### Example 7 (Gene Expression of Neurotrophic Factor in Dendritic Cells)

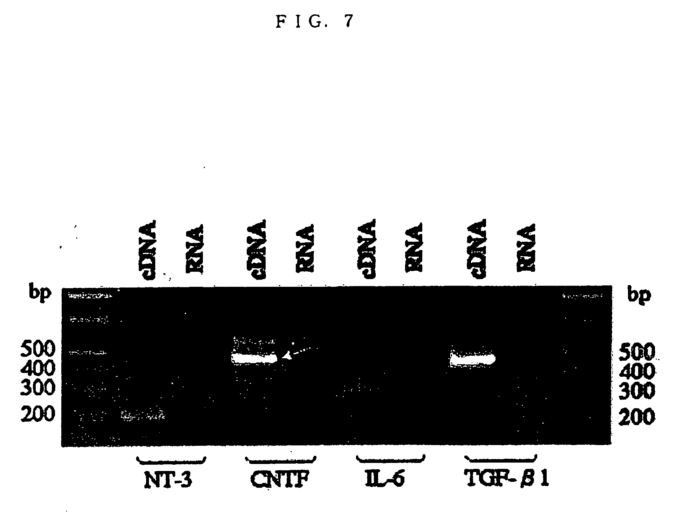

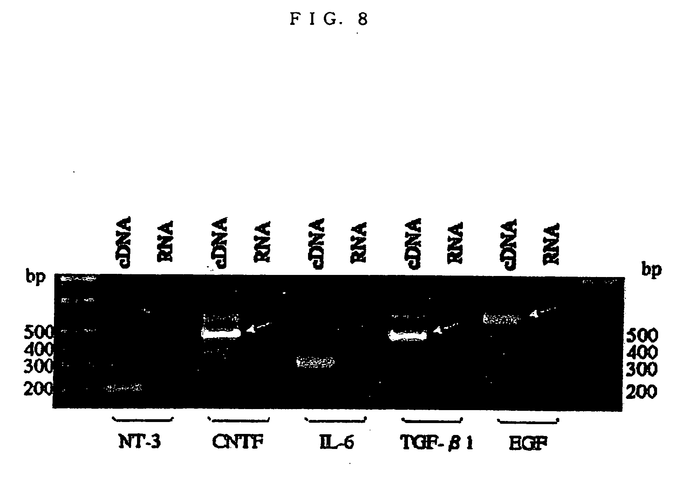

Total RNA was extracted from the cells of immature dendritic cell subsets and mature dendritic cell subsets with the use of TRIzol (Life Technologies), 5 µg each of total RNA was incubated at 42° C for 60 minutes by using AMV (Avian Myeloblastosis Virus) reverse transcriptase and an oligo (dT) primer, and a total amount of 200 µl cDNA was synthesized. PCR was conducted by using a primer of β-actin, gene expression was confirmed, and then PCR was conducted for each neurotrophic factor under respective conditions. PCR was conducted as follows : gene was amplified by using 1µl of cDNA as a template and a reaction enzyme of Extaq (TAKARA) and by a thermal cycler (Perkin-Elmer) . The primer used and the PCR condition are shown in Table 1. Besides, in order to show that it is not a gene product amplified from genomic DNA which was mixed in, PCR reaction was conducted respectively as a control by using total RNA as a template. The result in immature dendritic cell subsets is shown in Fig. 7, and the result in mature dendritic cell subsets is shown in Fig. 8, respectively.

**Table 1**

| | | Primer Sequence | |
|---|---|---|---|
| Identity | Size | Sense | Antisense |
| β -actin | 497 | 5'-CATGGCATTGTTACCAACTGG-3' (P1) | 5'-TGTGGTGGTGAAGCTGTAGC-3' (P2) |
| NT-3 | 200 | 5'-ACTACGGCAACAGAGACGCTAC-3'(P3) | 5'-ACAGGCTCTCACTGTCACACAC-3'(P4) |
| CNTF | 468 | 5'-TGGCTAGCAAGGAAGATTCGT-3'(P5) | 5'-ACGGAGGTCATGGATAGACCT-3'(P6) |
| IL-6 | 308 | 5'-TGCTGGTGACAACCACGGCC-3'(P7) | 5'-GTACTCCAGAAGACCAGAGG-3'(P8) |
| TGF-β1 | 462 | 5'-GAAGCCATCCGTGGCCAGAT-3'(P9) | 5'-GACGTCAAAAGACAGCCACT-3'(P10) |
| EGF | 595 | 5'-ACAGCCCTGAAGTGGATAGAG-3' (P11) | 5'-GGGCTTCAGCATGCTGCCTTG-3' (P12) |
| | | | |
| PCR Condition | | | |
| 94° C | 1 Min. | Thermal Denaturation | (However, β-actin; 30 Secs.) |
| 52° C | 1 Min. | Annealing | (However, β-actin; 63°C, NT-3; 65°C) |
| 72° C | 2 Mins. | Extension Reaction | (However, β-actin, NT-3: 1 Min.) |
| 42 cycles of the aforementioned thermal denaturation, annealing, and extension reaction. (However, β-actin; 30 cycles) | | | |

Expression of the following were confirmed in immature dendritic cells: NT-3 inducing the nerve regeneration effect in vivo, the proliferation of microglia, and the enhancement of phagocytosis; CNTF having the protective effect for denaturation and cell death against both motor and sensory neurons of the spinal cord; TGF-*β*1 having an inhibitory action for releasing cytotoxic substance derived from microglias and macrophages ; IL-6 inducing the protection effect against various neurons (cholin catecholamine dopaminergic) (Fig. 7). Besides, in mature dendritic cells, the expression of EGF wherein the nervous protection effect was recognized was confirmed in addition to NT-3, CNTF, TGF-β1, and IL-6 (Fig. 8). cDNA was extracted from the gel with regard to each gene, the base sequence was analyzed, and it was confirmed that the expression products were NT-3, CNTF, TGF-β1, IL-6 and EGF, respectively.

### Example 8 (Secretion of Neurotrophic Factor NT-3)

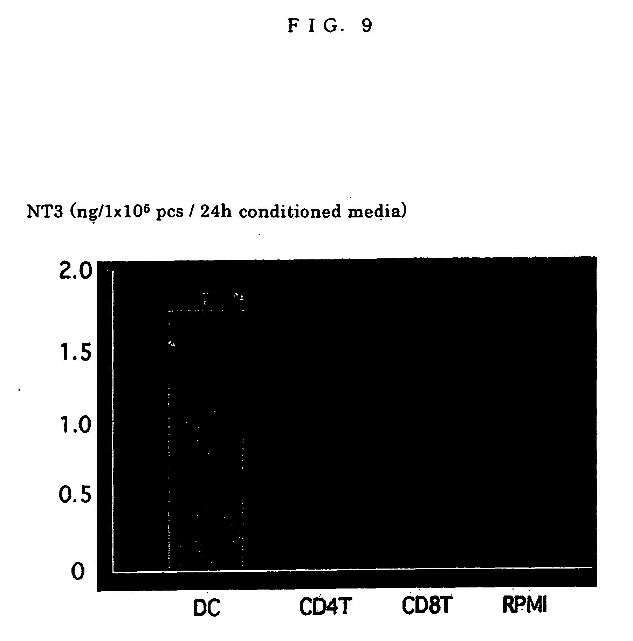

With regard to NT-3, one of the neurotrophic factor considered to be the most important for nerve regeneration, it was further analyzed whether it was actually secreted from dendritic cells by ELISA method using a NT-3 immunoassay system (Promega). CD11c positive immature dendritic cells were separated from the spleen of BALB/c female mature mice of 6 weeks old by the immune magnetic beads method in the same manner as in Example 1. After said 1 x 10⁵ of CD11c positive immature dendritic cells were incubated in a culture solution of RPMI1640 + 10% embryonic sera at 37°C under 5% CO₂ for 24 hours, its conditioned media were collected. Only RPMI1640 was used as a control, and 1 x 10⁵ of each CD4 positive T cells, CD8 positive T cells were used. As a result of quantitative analysis of NT-3 in the media by sandwich ELISA method wherein two types of anti NT-3 antibodies are used, it was revealed that 1 x 10⁵ of dendritic cells secreted approximately 1. 75 ng of NT-3 for 24 hours. When RPMI1640 only was used, and when CD4 positive T cells and CD8 positive T cells were used separately, secretion was not recognized (Fig. 9).

### Example 9 (Reconfirmation of the Effect of Dendritic Cells against a Spinal Cord Injury)

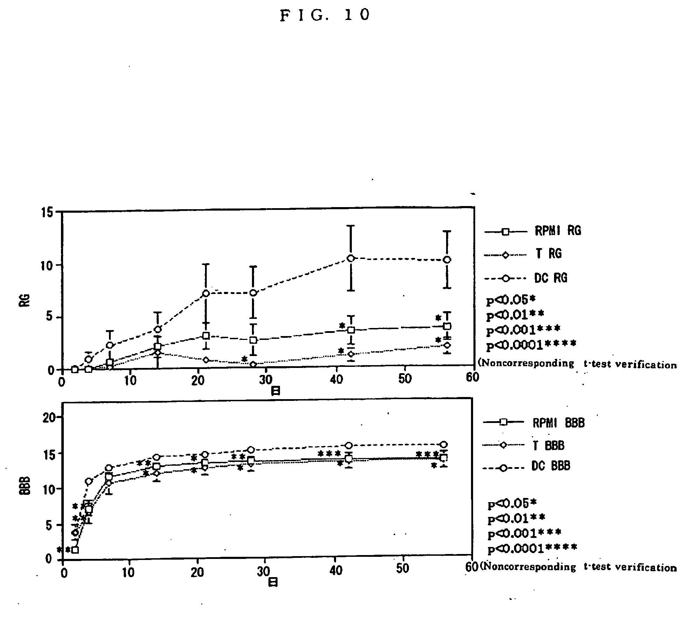

The eighth thoracic vertebra of the BALB/c female mice of 6 weeks old was laminectomized under ether anesthesia, and spinal cord injured model mice of which the left side of the spinal cord is cut in half under a microscope were generated. After transplanting 1 x 10⁶ of dendritic cells to the spinal cord injured site (DC, n=17) immediately, an evaluation was conducted chronologically by applying the motor function evaluation method for lower limbs developed by the present inventors (RG Score wherein the number of uprising motion is automatically analyzed by using the action analyzing apparatus, SCANET MV-10), and the BBB scale which is among the already established motor function evaluation method for lower limb (it is evaluated between 0 and 21 points, 0 point means that no lower limb motor is recognized, 21 points means normal.). RPMI1640 (RPMI, n = 18) and CD8 positive T cells (T, n = 10) were transplanted as a control to the spinal cord injured site in the same manner. The result is shown in Fig. 10 . As shown in Fig. 10 , DC transplanted group showed high scores of statistical significance in the both evaluation methods (RG Score and BBB scale) compared to the cases for T cell and RPMI of the controls. Accordingly, by transplanting dendritic cell subsets secreting neurotrophic factor NT-3 to the spinal cord injured site, it was reconfirmed that restoration of spinal cord function was promoted.

### Example 10 (Activation of Endogenous Microglias by Transplanting Dendritic Cells)

In order to examine whether any change by the transplant of dendritic cells can be seen in the reactivity of endogenous microglias or macrophages having invaded from the vein of the injured part, immunohistological staining was conducted by using Mac-1 antibodies which recognize them, and chronological change in the number of positive cells was investigated. Firstly, for the dendritic cell transplanted mice on day 2, 4, 7, and 14 after the injury, transcardiac perfusion fixation was conducted with 2% paraformaldehyde, and a cryosection was generated (n = 3). The RPMI1640 transplanted group was used as a control (n = 3). Secondly,immunohistologicalstaining wherein anti-mouse Mac-1 antibody (Pharmingen) was used as a primary antibody was conducted. Measuring region was divided into 3 parts i.e., the marginal injured site, cephalad aspect, and caudal aspect, as a portion covering from dorsal aspect to ventral aspect at each position of the most distal site of the gelfoam (denatured collagen) used for cell transplant and the site 1mm apart thereof. Types of Mac-1 positive cells to be measured were divided into two types, i.e. ameboid cells containing both of macrophages derived from monocytes flown from the outside of the spinal cord and activated macroglias wherein a phagocytic capacity is particularly strong, and ramified cells considered to be activated microglias lacking in phagocytic capacity.

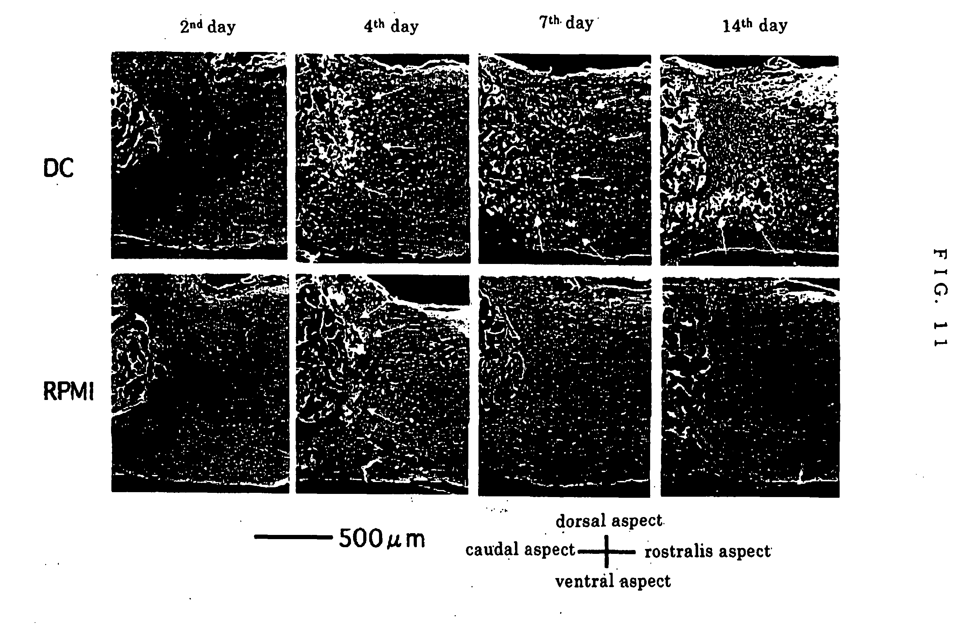

The staining image of a representative section covering from marginal injured site to cephalad aspect is shown in Fig. 11. In both groups, cellular infiltration is limited on day 2 after the injury, but distinguished infiltration of ameboid cell was recognized at the marginal injured site on day 4 after the injury. On and after day 4 after the injury, although infiltration of Mac-1 positive cell was recognized at cephalad distal part in the dendritic cell transplanted group, such change was limited in a control group.

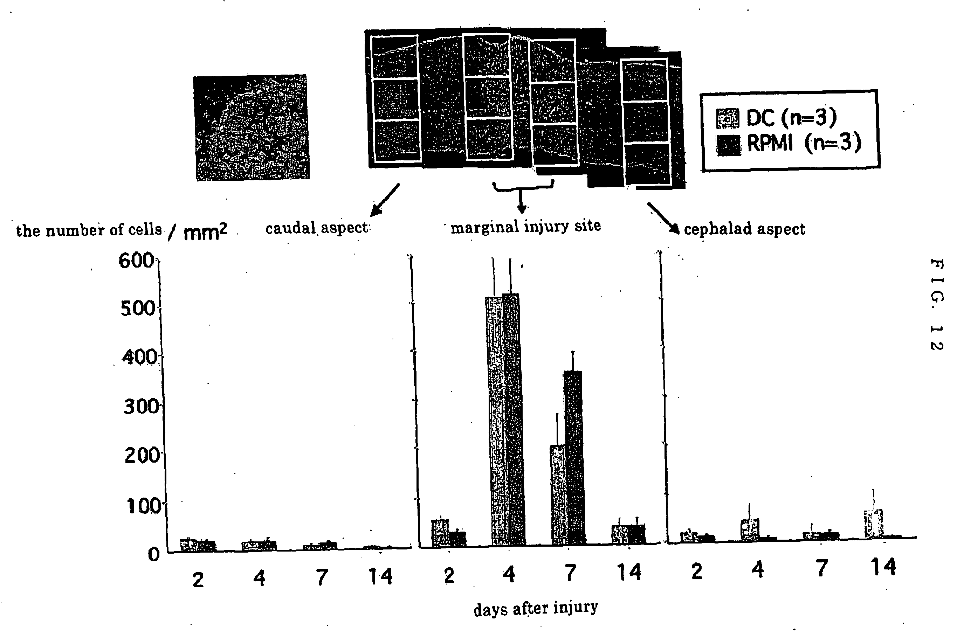

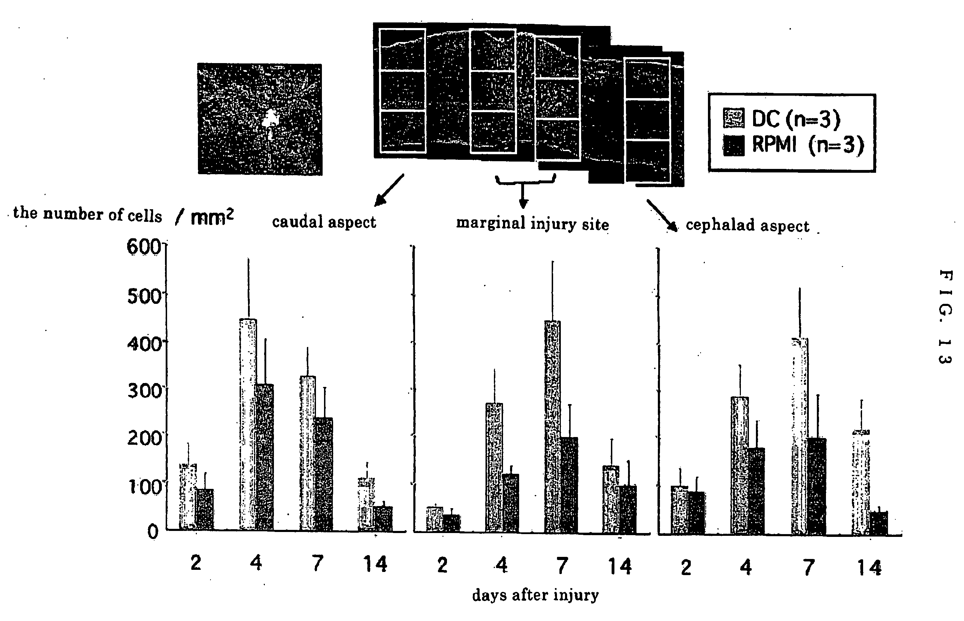

Subsequently, each Mac-1 positive cell was quantitatively analyzed respectively by using an image analysis apparatus (Flovel). Chronological change in the number of ameboid cells by each region is shown in Fig. 12. Infiltration of ameboid cells was mostly localized in the marginal injured site. Although obviously different number of cells between both groups at the marginal injured site or the caudal aspect was not recognized, a large number of positive cells was particularly recognized among dendritic cell transplanted group at the cephalad aspect on day 14 after the injury. On the other hand, Fig. 13 shows the chronological change in the number of ramified cells by each region, a larger number of cells was recognized in dendritic cell transplanted group in all regions, and on all measuring days . With regard to the fact that an ameboid activated microglia was increased in the cephalad aspect in the dendritic cell transplanted group, it is considered that since ameboid cells have a particularly strong phagocytic capacity, they are eliminating denatured myelin inhibiting the extension of a - nervous axon and a protein derived from injured tissue at a distant place from the injured site. On the other hand, since the increase of ramified activated microglias was seen in a wide range, it is considered that an activated microglia itself promoted the restoration of nervous function by secreting a neurotrophic factor such as NT-3, CNTF, IL-6, TGF-β1, EGF, bFGF, NGF, BDNF, GDNF and the like.

### Example 11 (Analysis of Endogenous Neural Stem Cells/Precursor Cells by Transplant of Dendritic Cells)

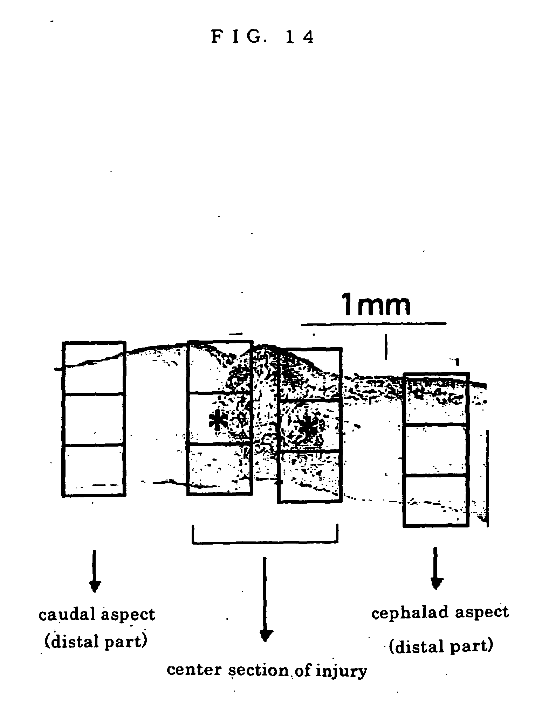

In order to examine the reactivity of endogenous neural stem cells/precursor cells by transplant of dendritic cells, immunohistological staining was conducted by using Musashi-1 antibody which recognize them, and chronological change in the number of positive cells was investigated. Firstly, for the dendritic cell transplanted mice of day 2, 4, and 7 after the injury, trans cardiac perfusion fixation was conducted with 2% paraformaldehyde, and a cryosection was generated (n = 3). The RPMI1640 transplanted group was used as a control (n = 3). Secondly, immunohistological staining wherein anti-mouse Musashi-1 antibody is used as a primary antibody was conducted. Musashi-1 is an RNA-bound protein of molecular weight approximately 38 kDa which was identified by Okano et al. in 1994 (Neuron, 1994), which was reported to strongly express in neural stem cells/precursor cells in the analysis using a monoclonal antibody against Musashi-1 of mouse (Dev. Biol. 1996, J. Neurosci. 1997, Dev. Neurosci. 2000). Measuring region was divided into 2 parts, i.e. the marginal injured site and the distal site (cephalad aspect and caudal aspect) as a portion covering from dorsal aspect to ventral aspect at each position of the most distal site of the gelfoam (denatured collagen) used for cell transplant and the site 1 mm apart thereof (Fig. 14).

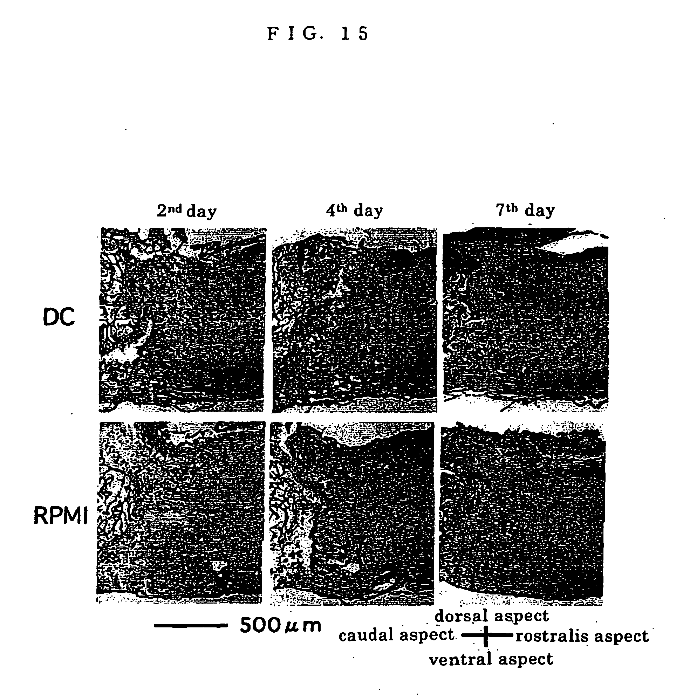

The staining image of a representative section covering from marginal injured site to cephalad aspect is shown in Fig. 15. In both groups, no difference can be seen on day 2 after the injury, however, on and after day 4 after the injury, a large number of Musashi-1 positive cells was recognized both in the marginal injured site and a distal site in the dendritic cells transplanted group, but such change was limited in a control group.

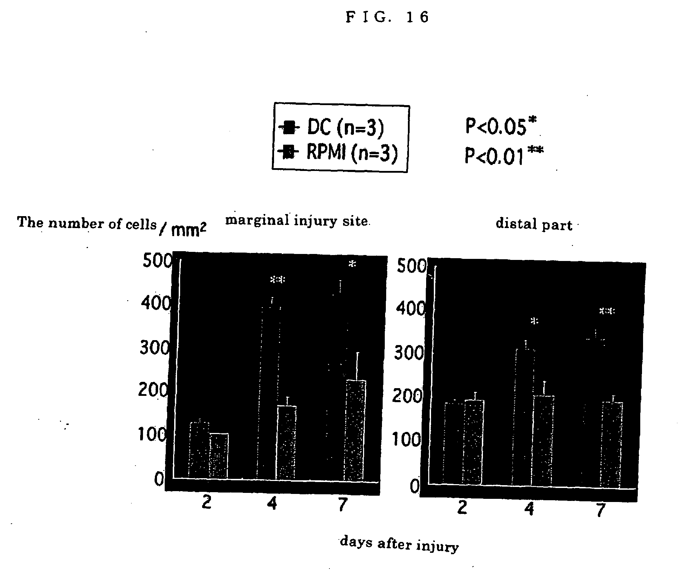

Subsequently, Musashi-1 positive cell was quantitatively analyzed by using an image analysis apparatus (Flovel). Chronological change in the number of Mushashi-1 positive cells by each region is shown in Fig. 16. More significant increase in the number of Musashi-1 positive cells was recognized by dendritic cells transplant both in the marginal injured site and a distal site on and after day 4 after the injury compared to a control. Particularly in the marginal injured site, significant increase of Musashi-1 positive cells was recognized in the dendritic cells transplanted group on day 2 to 4 after the injury.

As a result of the aforementioned, it was made clear that endogenous neural stem cells/precursor cells are induced to proliferate by the transplant dendritic cells.

### Example 12 (Expression Induction of Neurotrophic Factor in a Injured Neural Tissue after Administration of GM-CSF)

Spinal cord injured model mice were generated by using BALB/c female mice of 6 weeks old. 5 µl of physiological saline only or GM-CSF (250 pg/mouse; Genzyme) was administered to the spinal cord injured site immediately after the injury, and the spinal cord was extirpated on the second day. The spinal cord exterpated was frozen in liquid nitrogen, preserved at 80°C, and then total RNA was extracted by using TRIzol (Life Technologies). 5 µg each of total RNA was incubated at 42° C for 60 minutes by using AMV (Avian Myeloblastosis Virus) reverse transcriptase and oligo (dT) primer, and total amount of 200 µl cDNA was synthesized. PCR was conducted by using a primer of β-actin, gene expression was confirmed, and then PCR was conducted for each neurotrophic factor under each condition. PCR was conducted as follows: gene was amplified by using 1 µl of cDNA as a template and a reaction enzyme of Extaq (TAKARA) by a thermal cycler (Perkin-Elmer). The primer used and the PCR condition are shown in Table 2. Besides, in order to show that it is not a gene product amplified from genomic DNA which was mixed in, PCR reaction was conducted respectively as a control by using total RNA as a template.

**Table 2**

| | | Primer Sequence | |
|---|---|---|---|
| Identity | Size | Sense | Antisense |
| β -actin | 497 | 5'-CATGGCATTGTTACCAACTGG-3'(P1) | 5'-TGTGGTGGTGAAGCTGTAGC-3' (P2) |
| NT-3 | 200 | 5'-ACTACGGCAACAGAGACGCTAC-3' (P3) | 5'-ACAGGCTCTCACTGTCACACAC-3' (P4) |
| CNTF | 468 | 5'-TGGCTAGCAAGGAAGATTCGT-3' (P5) | 5'-ACGGAGGTCATGGATAGACCT-3' (P8) |
| BDNF | 277 | 5-CCAGCAGAAAGAGTAGAGGAG-3'(P13) | 5'-ATGAAAGAAGTAAACGTCCAC-3' (P14) |
| NGF | 498 | 5'-GTTTTGGCCTGTGGTCGTGCAG-3' (P15) | 5'-GCGCTTGCTCCGGTGAGTCCTG-3' (P16) |
| | | | |
| PCR Condition | | | |
| 94° C | 1 Min. | Thermal Denaturation | (However. β-actin; 30 Secs.) |
| 52° C | 1 Min. | Annealing | (However, β-actin; 63°C, NT-3; 65°C) |
| 72° C | 2 Mins. | Extension Reaction | (However, β-actin, NT-3; 1 Min.) |
| 35 cycles of the aforementioned thermal denaturation, annealing, and extension reaction. (However, β-actin; 30 cycles) | | | |

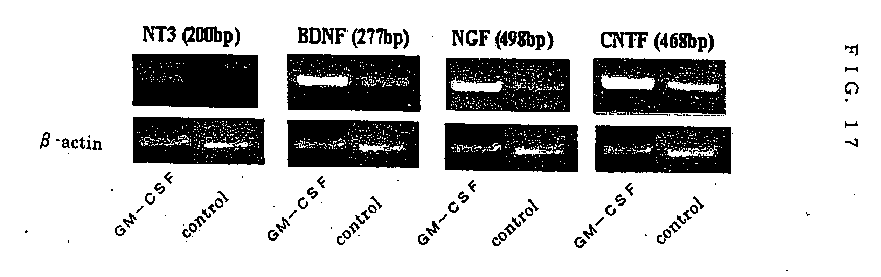

By administering GM-CSF to the injured spinal cord, it was revealed that expressions of the following are promoted: a neurotrophic factor, NT-3 inducing the nerve regeneration effect in vivo, the proliferation of microglia, and the enhancement of phagocytosis; a neurotrophic factor, BDNF inhibiting denaturation and omission of motor neuron of the injured spinal cord; a neurotrophic factor, NGF of cholinergic neuron; a neurotrophic factor, CNTF having protective effect for denaturation and cell death against both motor and sensory neurons of the spinal cord (Fig. 17).

### Example 13 (Activation of Endogenous Microglias by Administering GM-CSF)

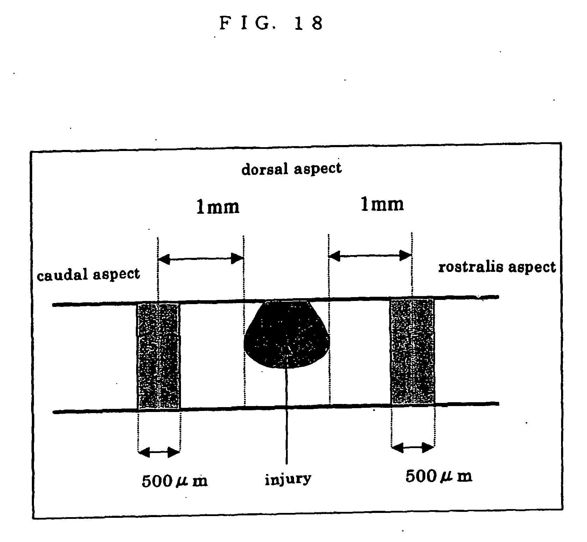

It is known that GM-CSF is involved in proliferation and activation of microglias and macrophages in vitro. In order to analyze its reactivity against microglias within a central nervous system tissue and macrophages having invaded from the vein of the injured part, immunohistological staining was conducted by using Mac-1 antibody which recognizes them, and chronological change in the number of positive cells was investigated. Firstly, for the GM-CSF administered mice of day 2, 4, and 7 after the injury, transcardiac perfusion fixation was conducted with 2% paraformaldehyde, and a cryosection was generated (n=3). Physiological saline-administered mice were used as control (n = 3). Secondly, immunohistological staining wherein anti-mouse Mac-1 antibody (Pharmingen) is used as a primary antibody was conducted. With regard to the measuring region, the region covering 1 mm apart to dorsal direction and ventral direction from the most distal part of the gelfoam (denatured collagen) used for cell transplant was analyzed (Fig. 18). Types of Mac-1 positive cells to be measured were divided in two types, i.e. an ameboid cell considered to be activated macroglias with a strong phagocytic capacity and macrophages derived from a monocytes flown from the outside of the spinal cord, and ramified cells considered to be activated microglias lacking in phagocytic capacity but secreting various neurotrophic factors and cytokines. They were quantitatively analyzed by using an image analysis apparatus (Flovel).

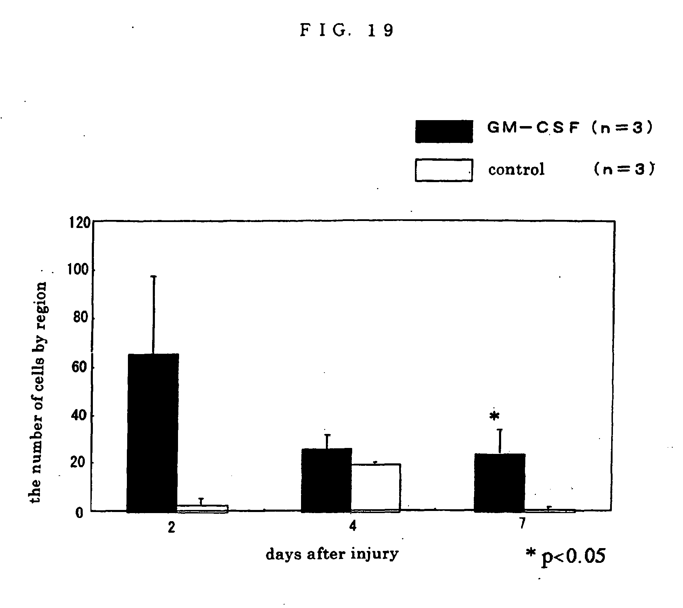

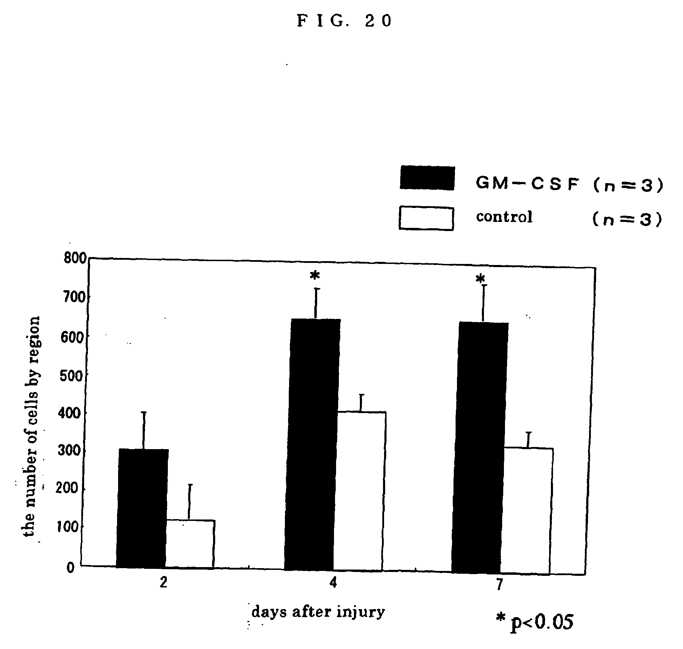

Chronological change in the number of ameboid cells and ramified cells is shown in Fig. 19 and Fig. 20, respectively. In the GM-CSF administered group, a large number of ameboid cells was recognized on day 2 after the injury, and significant increase in the number of cells compared to a control of day 7 after the injury was confirmed. And also in ramified cells, significant increases in the number of cells compared to a control were recognized on day 4 and 7 after the injury. With regard to the fact that ameboid cells were increased in GM-CSF administered group, it is considered that they are eliminating denatured myelin inhibiting the extension of a nervous axon and a protein derived from injured tissue since ameboid cells have particularly strong phagocytic capacity. In addition, the fact that the increase in the number of ramified cells was also seen indicates that activated microglia itself promoted restoration of the nervous function by secreting a neurotrophic factor such as NT-3, BDNF, NGF, CNTF and the like.

### Example 14 (Proliferation Induction of Endogenous Neural Stem Cells/Precursor Cells by Administering GM-CSF)

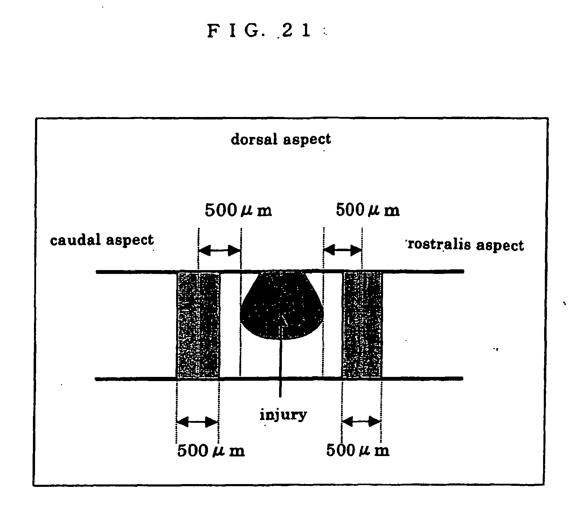

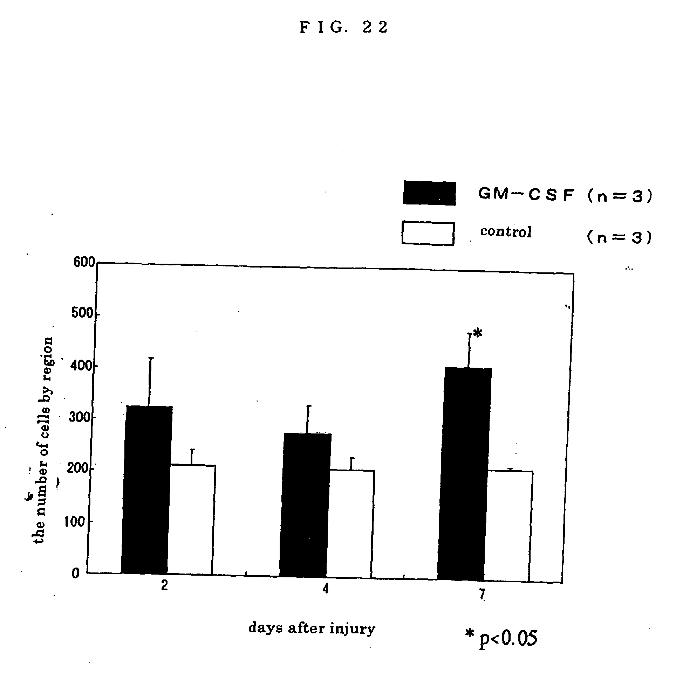

In order to analyze the reactivity against neural stem cells/precursor cells within the central nervous system by administering GM-CSF, immunohistological system staining was conducted by using Musashi-1 antibody that recognizes them, and chronological change in the number of positive cells was investigated. Firstly, for the GM-CSF administered mice of day 2, 4, and 7 after the injury, transcardiac perfusion fixation was conducted with 2% paraformaldehyde, and a cryosection was generated (n = 3). Physiological saline administered mice were used as a control (n = 3). Secondly, immunohistological staining wherein anti-Musashi-1 antibody (Pharmingen) is used as a primary antibody was conducted. With regard to the measuring region, the region covering 0 . 5 mm apart to dorsal and ventral direction from the most distal part the gelfoam (denatured collagen) used for cell transplanting was quantatively analyzed by using an image analysis apparatus (Fig. 21). Chronological change in the number of Musashi-1 positive cells is shown in Fig. 22. In GM-CSF administered group, a large number of Musashi-1 positive cells was recognized on and after day 2 from the injury compared to a control, and significant increase in the number of cells was recognized on day 7 from the injury. As aforementioned, it was revealed that endogenous neural stem cells/precursor cells are induced to proliferate by administering GM-CSF.

As a result of the aforementioned, it is considered that by way of transplanting dendritic cells to the injured site, nervous function was restored through the intermediaries of the following: direct secretion of a neurotrophic factor of its own, secretion of a neurotrophic factor through the activation of endogenous microglias, and the elimination action of inhibitor for the extension of a nervous axon, and regeneration and remyelination of a new neuron by proliferation induction of endogenous neural stem cells/precursor cells, and the like. It is also considered that by way of administering GM-CSF to the injured site, nervous function is restored through the intermediaries of the following: expression induction of a neurotrophic factor in a neuron, secretion of neurotrophic factor through activation of an endogenous microglia, and elimination action of inhibitor for extension of a nervous axon, and regeneration and remyelination of a new neuron by proliferation induction of endogenous neural stem cells/precursor cells, and the like.

### Industrial Applicability

The remedy for a nerve damage or a nerve dysfunctional disorder of the present invention can be administered not only by injecting into a injured site but also by various administration methods including subcutaneous administration or administration to a vicinity of lymph nodes, and intravenous administration, which has excellent nervous function restoration action, therefore, it is useful for the disorders of nerve dysfunctional disorders and the like such as a central nervous system injury including a spinal cord injury and a cerebral infarction. In addition, a dendritic cells related active substance such as IL-12, GM-CSF and the like are useful in that they can be easily handled and stored over a long time, and can be prepared in a large amount at any time, and can be applied to genetic therapies and the like.

## Claims

1. Use of interleukin-12 (IL-12) for preparing a remedy for nerve damage or nerve dysfunctional disorder wherein an IL-12 is used as an active ingredient.

2. The use of IL-12 for preparing a remedy according to claim 1 wherein the nerve damage or the nerve dysfunctional disorder is central nervous system damage or central nervous system dysfunctional disorder respectively.

3. The use of IL-12 for preparing a remedy according to claim 1 wherein the central nervous system damage or the central nervous system dysfunctional disorder is spinal cord injury or spinal cord dysfunctional disorder respectively.

4. Use of a vector which can express IL-12 for preparing a remedy for nerve damage or nerve dysfunctional disorder wherein the vector which can express IL-12 is used as an active ingredient.

5. The use of a vector which can express IL-12 for preparing a remedy according to claim 4 wherein the nerve damage or the nerve dysfunctional disorder is central nervous system damage or central nervous system dysfunctional disorder respectively.

6. The use of a vector which can express IL-12 for preparing a remedy according to claim 4 wherein the central nervous system damage or the central nervous system dysfunctional disorder is spinal cord injury or spinal cord dysfunctional disorder respectively.

## Patentansprüche

1. Verwendung von Interleukin-12 (IL-12) zur Herstellung eines Arzneimittels für Nervenschädigung oder Nervenfunktionsstörung, wobei ein IL-12 als ein aktiver Bestandteil verwendet wird.

2. Verwendung von IL-12 zur Herstellung eines Arzneimittels gemäß Anspruch 1, wobei die Nervenschädigung oder die Nervenfunktionsstörung jeweils eine Schädigung des zentralen Nervensystems oder eine Funktionsstörung des zentralen Nervensystems ist.

3. Verwendung von IL-12 zur Herstellung eines Arzneimittels gemäß Anspruch 1, wobei die Schädigung des zentralen Nervensystems oder die Funktionsstörung des zentralen Nervensystems jeweils eine Schädigung des Rückenmarks oder eine Funktionsstörung des Rückenmarks ist.

4. Verwendung eines Vektors, der IL-12 exprimieren kann, zur Herstellung eines Arzneimittels für Nervenschädigung oder Nervenfunktionsstörung, wobei der Vektor, der IL-12 exprimieren kann, als ein aktiver Bestandteil verwendet wird.

5. Verwendung eines Vektors, der IL-12 exprimieren kann, zur Herstellung eines Arzneimittels gemäß Anspruch 4, wobei die Nervenschädigung oder die Nervenfunktionsstörung jeweils eine Schädigung des zentralen Nervensystems oder eine Funktionsstörung des zentralen Nervensystems ist.

6. Verwendung eines Vektors, der IL-12 exprimieren kann, zur Herstellung eines Arzneimittels gemäß Anspruch 4, wobei die Schädigung des zentralen Nervensystems oder die Funktionsstörung des zentralen Nervensystems jeweils eine Schädigung des Rückenmarks oder eine Funktionsstörung des Rückenmarks ist.

## Revendications

1. Utilisation d'interleukine-12 (IL-12) pour la préparation d'un médicament pour une altération de nerfs ou un trouble dysfonctionnel de nerfs, dans laquelle une IL-12 est utilisée comme ingrédient actif.

2. Utilisation d'IL-12 pour la préparation d'un médicament suivant la revendication 1, dans laquelle l'altération de nerfs ou le trouble dysfonctionnel de nerfs est une altération du système nerveux central ou un trouble dysfonctionnel du système nerveux central, respectivement.

3. Utilisation d'IL-12 pour la préparation d'un médicament suivant la revendication 1, dans laquelle l'altération du système nerveux central ou le trouble dysfonctionnel du système nerveux central est une lésion de la moelle épinière ou un trouble dysfonctionnel de la moelle épinière, respectivement.

4. Utilisation d'un vecteur qui peut exprimer la IL-12 pour la préparation d'un médicament pour une altération de nerfs ou un trouble dysfonctionnel de nerfs, dans laquelle le vecteur qui peut exprimer la IL-12 est utilisé comme ingrédient actif.

5. Utilisation d'un vecteur qui peut exprimer la IL-12 pour la préparation d'un médicament suivant la revendication 4, dans laquelle l'altération de nerfs ou le trouble dysfonctionnel de nerfs est une altération du système nerveux central ou un trouble dysfonctionnel du système nerveux central, respectivement.

6. Utilisation d'un vecteur qui peut exprimer la IL-12 pour la préparation d'un médicament suivant la revendication 4, dans laquelle l'altération du système nerveux central ou le trouble dysfonctionnel du système nerveux central est une lésion de la moelle épinière ou un trouble dysfonctionnel de la moelle épinière, respectivement.
